# NEUE EUROPÄISCHE PATENTSCHRIFT

(11) **EP 1 328 242 B2**
(45) Veröffentlichungstag und Bekanntmachung des Hinweises auf die Entscheidung über den Einspruch: **22.07.2015**
(45) Hinweis auf die Patenterteilung: 02.11.2005
(21) Anmeldenummer: 02722218.1
(22) Anmeldetag: 14.03.2002
(51) Int. Cl.: A61K 8/44, A61K 8/49, A61Q 5/02, A61Q 5/04, A61Q 5/06, A61Q 5/10, A61Q 5/12

(54) **NICHT-THERAPEUTISCHE VERWENDUNG VON KREATIN, KREATININ UND/ODER DEREN DERIVATEN ZUR STÄRKUNG UND VERBESSERUNG DER HAARSTRUKTUR**
NON-THERAPEUTIC USE OF CREATINE, CREATININE AND/OR CYCLOCREATINE FOR STRENGTHENING AND IMPROVING THE STRUCTURE OF HAIR
UTILISATION NON-THERAPEUTIQUE DE LA CREATINE, DE LA CREATININE ET/OU DU CYCLOCREATINE AFIN DE RENFORCER LES CHEVEUX ET D'AMELIORER LEUR STRUCTURE

(30) Priorität: 24.03.2001 DE 10114561
(43) Veröffentlichungstag der Anmeldung: 23.07.2003
(73) Patentinhaber: Wella Aktiengesellschaft, 64274 Darmstadt (DE)
(72) Erfinder: BIMCZOK, Rudolf, 64342 Seeheim-Jugenheim (DE); KRIPP, Thomas, 64407 Fränkisch-Crumbach (DE); GRASSER, Beate, 65795 Hattersheim (DE); SPRINGOB, Christian, 64625 Bensheim (DE)
(74) Vertreter: Boubel, Thomas
(86) Internationale Anmeldenummer: PCT/EP2002/002839
(87) Internationale Veröffentlichungsnummer: WO 2002/076408

(56) Entgegenhaltungen:
- EP-A- 0 565 010
- EP-A1- 0 413 528
- EP-A1- 0 880 916
- EP-A1- 1 166 762
- WO-A-01/00203
- WO-A-02/02075
- WO-A1-00/15187
- WO-A1-98/22087
- WO-A1-03/005988
- WO-A2-01/01932
- WO-A2-02/060394
- DE-A- 3 709 459
- DE-A- 3 941 534
- DE-A- 19 841 385
- DE-A1- 3 138 142
- DE-C- 348 194
- FR-A- 2 725 896
- JP-A- H1 135 425
- JP-A- H09 194 333
- JP-K1- H1 135 425
- US-A- 6 159 485
- PATENT ABSTRACTS OF JAPAN vol. 018, no. 632 (C-1280), 2. Dezember 1994 (1994-12-02) & JP 06 240579 A (LION CORP), 30. August 1994 (1994-08-30)
- DATABASE PROMT [Online] STN ; AN 2002:414055, "Natural ingredients" XP002218034 & HOUSEHOLD & PERSONAL PRODUCTS INDUSTRY, Bd. 39, Nr. 6, Juni 2002 (2002-06), Seite 76 (8)
- JP H09194333 A PAJ ZUSAMMENFASSUNG
- JP H09194333 A CA ZUSAMMENFASSUNG
- JP H09194333 A WPI ZUSAMMENFASSUNG
- JP H09194333 A ZUSAMMENFASSUNG
- JP H1135425 A PAJ ZUSAMMENFASSUNG
- JP H1135425 A, CA ZUSAMMENFASSUNG
- JP H1135425 A, WPI ZUSAMMENFASSUNG
- 'Der Grosse Brockhaus', Bd. 5, 1954, F.A. BROCKHAUS, WIESBADEN Seite 4460
- 'Das Haar und seine Struktur', Bd. 1, 1999, WELLA AG, DARMSTADT Seiten 34 - 38

## Beschreibung

Gegenstand der vorliegenden Erfindung ist die nicht-therapeutische Verwendung von Kreatin, Kreatinin und/oder deren Salzen zur Härtung, Stärkung und Strukturverbesserung (Restrukturierung) von geschädigten menschlichen Haaren.

Die Schädigung von keratinischen Fasern durch Umwelteinflüsse (beispielsweise energiereiche Strahlungen), den physiologischen Status (beispielsweise Alter oder Gesundheit der betreffenden Individuen) oder mechanische und chemische Einwirkungen sind bekannt. Die Folgen sind nachteilige mechanische Eigenschaften der betroffenen Materialien. Derartige Schädigungen der inneren Struktur von keratinischen Fasern zeigen sich zum Beispiel durch Verlust an Härte, Glanz, Stärke, Bruchfestigkeit, Reißfestigkeit oder Bündelzugfestigkeit.

An Keratinfasern, besonders an menschlichen Haaren, machen sich solche Einflüsse insbesondere durch fehlenden Glanz, verminderte Reißkraft und schlechte Kämmbarkeit bemerkbar. Hervorgerufen werden sie durch Alterungsprozesse, die vor allem physiologisch bedingt sind oder durch physikalische (Bewitterung), mechanische (Kämmen, Bürsten) und chemische Einflüsse induziert werden. Bei längeren Haaren machen sich diese Einflüsse insbesondere in den Haarspitzen bemerkbar. Zu den chemischen Einflüssen gehören vor allem das Bleichen, oxidatives Färben und Dauerwellen der Haare, wobei aggressive Oxidations- bzw. Reduktionsmittel zudem bevorzugt in stark alkalischem Milieu angewendet werden und dort ihre volle Wirkung zeigen. Aber auch andere chemische Einflüsse entfalten schädigende Wirkungen auf Keratin enthaltendes Material, beispielsweise mit Chlor oder Salzen angereichertes Wasser.

Handelsübliche Spülungen und Kuren enthalten als Aktivsubstanzen hauptsächlich kationische Tenside bzw. Polymere, Wachse und/oder Öle. Je geschädigter das Haar ist, desto mehr anionische Gruppen finden sich an der Oberfläche. Kationische Verbindungen werden auf dieser entgegengesetzt geladenen Oberfläche elektrostatisch angezogen, während Öle und Wachse mit den hydrophoben Gruppen des Keratins wechselwirken. Eine Strukturverbesserung im Haarinneren lässt sich mit diesen Pflegeprodukten daher nicht erreichen.

Der Einsatz von bestimmten ungesättigten Verbindungen, insbesondere Ascorbinsäure, in Haarbehandlungsmitteln für diesen Zweck ist aus der eigenen WO00/57839 bekannt. Ascorbinsäure ist jedoch in wässriger Lösung nicht lange beständig, so dass solche Mittel nicht gelagert werden können, sondern erst kurz vor der Anwendung hergestellt werden müssen.

Aus der DE 198 41 385 A1 ist die Verwendung von Kreatin und Kreatinderivaten als Feuchthaltemittel für kosmetische Zubereitungen bekannt. Die Zubereitungen sollen die elastischen Eigenschaften der Haut verbessern sowie zur Faltenglättung und Beseitigung von Rissen und Schuppen beitragen.

Die EP 0 565 010 A1 offenbart, dass Levodopa neues Haarwachstum aktiviert und die Haarrepigmentierung fördert. Es ist wirksam, indem es direkt auf die Kopfhaut aufgetragen wird und die lokale Mikrozirkulation des Blutes aktiviert, indem es die Blutgefäße erweitert. Ein Zusatz eines Phosphorsäuresalzes, wie z. B. Phosphokreatin, soll diese Wirkungen synergistisch verstärken.

In der WO 01/00203 A1 ist ein Verfahren zur Behandlung (Vorbeugung, Verringerung oder Verbesserung) von Beeinträchtigungen der Haut beschrieben, bei dem eine wirksame Menge Kreatin, Kreatinphosphat oder eine Kreatinverbindung oder deren Salz auf die Haut aufgebracht wird. Die Beeinträchtigungen sind insbesondere Faltenbildung durch Stress, Sonnenbestrahlung, Ermüdung und/oder freie Radikale.

Aus den Patent Abstracts of Japan zu JP 06-240579 A1 sind Mittel zur Behandlung von Keratinfasem wie Haaren zwecks Desodorierung und Erzielung eines Geruchsvermeidungseffekts bekannt. Als deodorierendes Agens sollen 0,01 bis 5 Gew.% eines Deodorans vom Aminosäuretyp, vorzugsweise Kreatin bzw. Kreatinin, enthalten sein.

In der DE 39 41 534 A1 wird ein Haarkonditionierungsmittel beschrieben, das ein Guanidinderivat mit einer Aminogruppe im Molekül oder ein Salz davon als Wirkstoff enthält. Es soll geschädigtem Haar eine weiche und feuchte Beschaffenheit geben .

Die der vorliegenden Erfindung zu Grunde liegende Aufgabe bestand darin, ein Mittel, insbesondere ein kosmetisches Haarbehandlungsmittel, für die nicht-therapeutische Verwendung zur Verbesserung des Haarzustandes bereitzustellen, das die vorgenannten Nachteile beseitigt.

Erfindungsgemäß wird diese Aufgabe gelöst durch die nicht-therapeutische Verwendung von Kreatin, Kreatinin und/oder deren Salze zur Härtung, Stärkung, und Restrukturierung von geschädigten menschlichen Haaren, wobei die Verwendung die Nasskämmbarkeit der Haare erhöht.

Überraschenderweise wurde nämlich gefunden, dass durch die Verwendung von Kreatin, Kreatinin und/oder deren Salzen die Struktur von keratinischen Fasern (Haaren) derartig verändert wird, dass eine Härtung und Stärkung sowie die Erhöhung der Bruchfestigkeit, Reißfestigkeit oder Bündelzugfestigkeit, insbesondere der strapazierten der geschädigten Haare erfolgt.

Neben einer Pflegewirkung, die aus der Beeinflussung der Haaroberfläche (Cuticula) resultiert, zeigte sich somit insbesondere eine Repair-Wirkung. Diese geht auf Veränderungen im Inneren des Haares (Cortex) zurück. Es wurden die Zugkräfte gemessen, die oxidativ (durch Bleichen) vorgeschädigte Haare zum Zerreißen bringen. Überraschend wurde gefunden, dass diejenigen Haare, die nach der oxidativen Schädigung mit einem Kreatin, Kreatinin und/oder deren Salze enthaltenden Mittel behandelt worden waren, eine signifikante Erhöhung der zum Reißen notwendigen Kräfte aufwiesen.

Dies ist überraschend, da aufgrund der Struktur des Kreatins und dessen Derivate weder das Eindringen ins Haarinnere, noch überhaupt eine Beeinflussung der Proteinstrukturen vorhersehbar war. Zudem ist bekannt, dass ungeschädigte nasse Haare deutlich geringere Reißkräfte haben (600 - 900) mN als trockene (1000 - 1500) mN. Somit sollte man erwarten, dass ein Feuchthaltemittel wie Kreatin bzw. Kreatinin die Reißkräfte eher senkt, statt diese, wie gefunden, zu erhöhen.

Damit verbunden wird nicht nur eine Restrukturierung (Repair) von geschädigten keratinischen Fasern ermöglicht, sondern auch ein protektiver Effekt, der einer Schädigung dieser Materialien vor oder während einer Exposition mit entsprechenden Noxen entgegenwirkt und sie zu verhindern oder zu vermindern vermag.

Neben diesen von durch exogene Noxen hervorgerufenen nachteiligen Veränderungen kann die erfindungsgemäße Verwendung auch bei durch physiologische Prozesse bedingte Zustände oder Änderungen der Struktur von keratinischen Fasern vorteilhafte Wirkungen entfalten; beispielsweise bei altersbedingtem brüchigem Haar oder bei feinem Haar, welches angeboren oder altersabhängig erworben sein kann (Babyhaar, Altershaar).

Damit verbunden konnte ferner festgestellt werden, dass bei Haaren, durch die erfindungsgemäße Verwendung eine Volumenerhöhung erreicht werden kann, was sich vorteilhaft bei der Herstellung von Frisuren (Erhöhung der Haarfülle) auswirken kann. Es wird vermutet, dass die Wirkung der Volumenerhöhung in kausalem Zusammenhang mit der haarhärtenden, haarstärkenden bzw. haarrestrukturierenden Wirkung der erfindungsgemäß verwendeten Mittel steht. Dies war überraschend, da üblicherweise Substanzen, die eine Feuchthaltewirkung besitzen, das Haarvolumen eher verringern.

Gegenstand der vorliegenden Erfindung ist somit die nicht-therapeutische Verwendung gemäß Anspruch 1.

Die verwendeten Stoffe, das Kreatin (N-Amidinosarcosin) (Formel I) sowie sein Zyklisierungsprodukt Kreatinin (2-Imino-1-methylimidazolidin-4-on) (Formel II) sind physiologischer Bestandteil menschlichen und tierischen Gewebes.

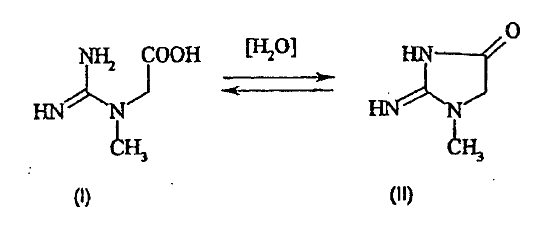

Weitere besonders bevorzugt verwendete Substanzen sind Kreatin-phosphat, Cyclokreatin, Phosphocyclokreatin und Kreatin-pyruvat.

Es können auch beliebige Gemische des Kreatins, des Kreatinins und/oder deren Salze für den genannten Zweck verwendet werden.

Weitere Ausführungsformen der vorliegenden Erfindung sind in den Ansprüchen wiedergegeben.

Die nicht-therapeutische Verwendung erfolgt, indem die Haare mit einem das Kreatin, das Kreatinin und/oder deren Salze enthaltenden Mittel in Kontakt gebracht werden und dieses nach Applikation vorzugsweise dort verbleibt oder nach einer geeigneten Einwirkungszeit mit einem wässrigen Mittel ab- oder ausgespült wird.

Bevorzugt ist es, wenn das Kreatin, das Kreatinin und/oder deren Salze in einer Menge von 0,001 bis 30,0 Gewichtsprozent, bevorzugt 0,01 bis 10,0 Gewichtsprozent, besonders bevorzugt 0,05 bis 1,0 Gewichtsprozent, jeweils bezogen auf die Gesamtmenge des Mittels, in dem Mittel enthalten ist.

Das für die erfindungsgemäße Verwendung beschriebene Mittel kann in allen geeigneten Formulierungen vorliegen, die in der kosmetischen oder pharmazeutischen Industrie bekannt sind. Insbesondere kann das Mittel als wäßrige oder wäßrig-alkoholische Lösung, als Gel, Creme, Emulsion oder Schaum vorliegen, wobei das Mittel sowohl in Form eines Einkomponentenpräparats als auch in Form eines Mehrkomponentenpräparates konfektioniert sein kann. Im Falle eines Einkomponentenpräparates enthält das Mittel Kreatin, Kreatinin und/oder Salze davon zusammen mit geeigneten Hilfs- und Trägerstoffen (beispielsweise Verdickern, Säuren, Duftstoffen, Lösungsmitteln, Salzen, Netzmitteln und/oder UV-Absorbern).

Liegt das Mittel in Form eines Mehrkomponentenpräparates vor, kann dieses aus mindestens zwei verschiedenen, bis zur nicht-therapeutischen Anwendung voneinander räumlich getrennten Komponenten bestehen. Eine erste Komponente kann entweder das der vorliegenden Erfindung zugrundeliegende Kreatin, Kreatinin und/oder Salze davon (Wirkstoff) für sich allein enthalten oder der Wirkstoff kann zusammen mit einem Hilfsstoff (zum Beispiel ein Verdickungsmittel), vorteilhafterweise in fester trockener Form (zum Beispiel als Pulver in verpresster oder nicht-verpresster Form, als Granulat oder Tablette), vermischt in dieser ersten Komponente vorliegen. Eine zweite oder weitere Komponente enthält nur Hilfs- und Trägerstoffe.

Es ist aber auch möglich, dass in einem Mehrkomponentenpräparat verschiedene Komponenten verschiedene Wirkstoffe gemäß der vorliegenden Erfindungen einzeln oder als Gemisch enthalten, entweder für sich allein oder zusammen mit verschiedenen Hilfsstoffen vermischt und das die weiteren Komponenten nur Hilfs- und Trägerstoffe enthalten.

Die erfindungsgemäße Verwendung kann erfolgen mit einer Zusammensetzung, die dadurch gekennzeichnet ist, dass sie als Einkomponentenpräparat oder als Mehrkomponentenpräparat vorliegt. Liegt das verwendete Mittel als Mehrkomponentenpräparat vor, so umfasst es eine erste Komponente, die das Kreatin, Kreatinin und/oder deren Salze mit oder ohne Hilfs- und Zusatzstoffe enthält und eine zweite Komponente, welche die übrigen Bestandteile enthält. Das verwendete Mittel kann ferner als Mehrkomponentenpräparat mit mindestens drei verschiedene Komponenten vorliegen, wobei mindestens eine der Komponenten Kreatin, Kreatinin und/oder deren Salze enthält und die übrigen Komponenten die restlichen Bestandteile enthalten.

Es ist selbstverständlich, dass zur Herstellung eines gebrauchsfertigen Mittels die räumlich getrennt vorliegenden einzelnen Komponenten einer Mehrkomponentenpräparation kurz vor deren erfindungsgemäßen Verwendung vermischt werden müssen.

Das erfindungsgemäß verwendete Mittel kann zusätzlich Träger- und Hilfsstoffe, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol; weiterhin Lösungsvermittler, Netzmittel oder Emulgatoren aus den Klassen der anionischen, kationischen, amphoteren oder nichtionogenen oberflächenaktiven Substanzen wie Fettalkoholsulfate, ethoxylierte Fettalkoholsulfate, Alkylsulfonate, Alkylbenzolsulfonate, Alkyltrimethylammoniumsalze, Alkylbetaine, ethoxylierte Fettalkohle, ethoxylierte Nonylphenole, ethoxylierte Fettalkohole, ethoxylierte Nonylphenole, Fettsäurealkanolamide, ethoxylierte Fettsäureester, ferner Verdicker wie höhere Fettalkohole, Stärke oder Cellulosederivate; Salze wie z. B. NaCl; Puffersubstanzen wie Ammoniumhydrogencarbonat; Thiole, Ketocarbonsäuren (Oxocarbonsäuren), insbesondere α-Ketocarbonsäuren, bzw. deren physiologisch verträgliche Salze, UV-Absorber, Parfüme, Farbstoffe, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain; Treibmittel wie z. B. Propan, Butan, Dimethylether, N₂O und Kohlendioxid, enthalten.

Die vorstehend erwähnten Bestandteile werden in den für solche Zwecke üblichen Mengen verwendet, zum Beispiel Wasser in einer Menge von 0,1 bis 95 Gew.%, die Netzmittel und Emulgatoren in Konzentrationen von insgesamt 0,2 bis 30 Gewichtsprozent, die Alkohole in einer Menge von insgesamt 0,1 bis 50 Gewichtsprozent, die Trübungsmittel, Parfümöle, Konservierungsstoffe und Farbstoffe in einer Menge von jeweils 0,01 bis 5 Gewichtsprozent, die Puffersubstanzen in einer Menge von insgesamt 0,1 bis 10 Gewichtsprozent, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Der pH-Wert des Mittels beträgt bevorzugt 2,0 bis 10,0, insbesondere 3,0 bis 9,0. Erforderlichenfalls kann der gewünschte pH-Wert durch Zugabe von Säuren, beispielsweise α-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Ammoniak, Alkanolaminen, Alkylaminen, Alkalihydroxiden, Ammoniumhydroxiden, Alkalicarbonaten, Ammoniumcarbonaten oder Alkaliphosphaten, eingestellt werden.

Bei der nicht-therapeutischen Behandlung menschlicher Haare kann das Mittel im Haar verbleiben oder nach der nicht-therapeutischen Anwendung ausgespült werden. Im letzteren Fall beträgt die Einwirkungszeit des Mittels, je nach Temperatur (etwa 20 bis 60 Grad Celsius, vorzugsweise 30 bis 50 Grad Celsius), 1 bis 60 Minuten, insbesondere 5 bis 20 Minuten, wobei durch Wärmezufuhr die Repairwirkung (Härtung, Restrukturierung und gegebenenfalls die damit verbundene Volumenerhöhung) beschleunigt werden kann; daher ist die Anwendung von Wärme bevorzugt. Nach Beendigung der Einwirkungszeit kann das Haar mit Wasser gespült und gegebenenfalls mit einem Shampoo gewaschen werden.

Bei den für die erfindungsgemäße Verwendung geeigneten Konfektionierungen des Mittels handelt es sich vorzugsweise um Shampoos, Spülungen, Kuren, Schäumen, Festigern, Haargelen, Haarsprays, Haarfarben, Haartönungen, Dauerwellmitteln, Fixierungen, Haarglättungsmitteln oder Brillantinen.

Das Mittel kann auch als Vorbehandlungsmittel vor chemischen und/oder physikalischen Behandlungen von keratinischen Fasem, insbesondere einer Haarfärbung, einer Haartönung, einer Haarbleichung oder vor einer Haardauerverformung, nicht-therapeutisch verwendet werden, um einer Haarschädigung durch diese oxidativen Behandlungen vorzubeugen.

Es konnte festgestellt werden, dass die erfindungsgemäße Verwendung von Kreatin, Kreatinin und/oder deren Salze gemäß der vorliegenden Erfindung eine deutliche Strukturverbesserung zuvor geschädigter keratinischer Fasern ermöglicht, die sich zudem mit einer statistisch hochsignifikanten Erhöhung der Reißkraft nachweisen lässt.

### Messung der Repairwirkung

Die Ermittlung der Reißkraft von Haaren, die ein Indikator für die strukturelle Integrität des Haarcortex und damit ein Maß für den Schädigungsgrad ist, erfolgt durch für diese Zwecke übliche Zug-Dehnungs-Messungen. Von jeder Haarsträhne werden 20 Einzelhaare ausgewählt und die einzelnen Haardurchmesser mit einem computergesteuerten Lasermikrometer bestimmt. Anschließend wird mit einem Zug-Dehnungs-Meßgerät (MTT 160/600 Series Miniature Tensile Tester, Serial No. 600.95.05.001, Fa. DIA-STRON® Ltd., England) die Kraft gemessen, die nötig ist, um die einzelnen Haare zum Zerreißen zu bringen.

Aus diesen einzelnen, aufgrund der unterschiedlichen Haardurchmesser voneinander abweichenden Reißkraft-Meßwerten wird die sog. Bündelzugfestigkeit (BZF) ermittelt, indem aus den Einzelwerten unter Berücksichtigung der jeweiligen Haardurchmesser die Reißkraft für einen Haardurchmesser von 0,08 mm (mittlerer Durchmesser) errechnet wird. Durch Einbeziehung der Haardichte erfolgt schließlich die Umrechnung in die Einheit der Bündelzugfestigkeit (cN/tex). Je größer der Zahlenwert der Bündelzugfestigkeit, desto geringer ist die Haarschädigung.

Die Messungen an Haaren, die mit kreatinhaltigen und kreatinfreien Shampoos behandelt wurden, ergaben folgende Ergebnisse:
Geschädigtes Haar (blondiert) wurde behandelt mit dem Shampoo nach Beispiel 2 jedoch ohne Kreatin (die Menge an Kreatin wurde durch Wasser ersetzt): BZF =14,8 +/ 0,4 cN/tex (ermittelt an 17 Haaren aus einer Shampoo-behandelten Strähne);
Geschädigtes Haar (blondiert) behandelt mit dem Shampoo nach Beispiel 2 mit 1 Gew.% Kreatin: BZF = 15,5 +/- 0,4 cN/tex (ermittelt an 19 Haaren aus einer Shampoo-behandelten Strähne).

Der Unterschied zwischen den oben angegebenen Mittelwerten ist statistisch hochsignifikant (Signifikanzniveau ermittelt mit t-Test: 99,9%).
Die Bündelzugfestigkeit wird durch den Gehalt an 1 Gew.% Kreatin von 14,8 cN/tex auf 15,5 cN/tex erhöht; dies entspricht einem Anstieg um 4,7 %.
Der Zusatz von Kreatin bedingt somit eine deutliche Haarstärkung bzw. Repairwirkung.

### Messung der Kämmbarkeitsverbesserung

Die Kämmbarkeit des Haares ist ebenfalls ein wichtiger Parameter zur Beschreibung der Haarqualität. Verschiedene äußere Einwirkungen wie bestimmte kosmetische Behandlungen (Bleichen, Färben, Dauerwellen), Bewetterung, häufiges Kämmen und Bürsten verschlechtern die Kämmbarkeit des Haares, was auf eine Schädigung der Cuticula zurückzuführen ist.

Das Prinzip der meisten Verfahren zur messtechnischen Erfassung der Kämmbarkeit besteht darin, die Kraft (Kämmkraft) zu messen, die nötig ist, um einen Kamm unter exakt definierten Randbedingungen durch eine Haarsträhne zu kämmen.

Im Rahmen der eigenen Untersuchung wurde dazu eine automatisierte Apparatur verwendet, bei der ein mechanischer Greifarm die zu untersuchenden Strähnen aus einem Lager holt und auf den Haken einer Kraftmessdose hängt. Dann werden die Strähnen nacheinander mehrmals automatisch mit konstanter Geschwindigkeit gekämmt und für jeden Kämmvorgang wird die Kämmkraft N (Newton) als Funktion der Kämmstrecke (Strähnenlänge) aufgenommen. Die angegebenen Kämmkraftwerte ergeben sich schließlich aus einer Mittelwertbildung der Kämmkräfte über die Kämmstrecke. Je geringer die Kämmkraft ist, desto besser ist die Kämmbarkeit des Haares.

Die Messungen an Haaren, die mit kreatinhaltigen und kreatinfreien Shampoos behandelt wurden, ergaben folgende Ergebnisse:
Geschädigtes Haar (blondiert) wurde behandelt mit dem Shampoo nach Beispiel 2 jedoch ohne Kreatin (die Menge an Kreatin wurde durch Wasser ersetzt):
   Die Kämmkraft betrug 1,43 +/- 0,05 N (gemessener Mittelwert aus 3 mit Shampoo behandelten Haarsträhnen).
Geschädigtes Haar (blondiert) behandelt mit Shampoo nach Beispiel 2 mit 1 Gew.% Kreatin:
   Die Kämmkraft betrug 1,21 +/- 0,08 N (gemessener Mittelwert aus 3 mit Shampoo behandelten Haarsträhnen).
Der Unterschied zwischen den oben angegebenen Mittelwerten ist statistisch signifikant (Signifikanzniveau ermittelt mit t-Test: 97,5%).

Die Kämmkraft verringert sich durch den Gehalt an 1 Gew.% Kreatin von 1,43 N auf 1,21 N, also um 15,3 %. Der Zusatz von Kreatin bedingt somit eine deutlich feststellbare Verbesserung der Nasskämmbarkeit des Haares.

Alle in der vorliegenden Beschreibung genannten Prozentangaben stellen, soweit nicht anders angegeben, Gewichtsprozente, bezogen auf das Gesamtgewicht der jeweiligen Zusammensetzung, dar.

Die nachfolgenden Beispiele sollen den Gegenstand näher erläutern.

### Beispiele

| Beispiel 1: Haarspray | |
|---|---|
| Vinylacetat/Crotonsäure-Copolymer | 2,00 Gew.% |
| 2-Amion-2-methyl-1-propanol | 0,16 Gew.% |
| Ethanol | 37,84 Gew.% |
| Kreatin | 0,50 Gew.% |
| Parfümöl | 0,10 Gew.% |
| Propan/Butan | ad 100,00 Gew.% |

| Beispiel 2: Haarshampoo | |
|---|---|
| Natriumlaurylethersulfat (25%ige wässrige Lösung) | 40,0 Gew.% |
| NaCl | 4,0 Gew.% |
| Kreatin | 1,0 Gew.% |
| Wasser | ad 100,0 Gew.% |

| Beispiel 3: Haarshampoo | |
|---|---|
| Natriumlaurylethersulfat (25%ige wässrige Lösung) | 35,0 Gew.% |
| NaCl | 3,0 Gew.% |
| Triethanolamin | 4,0 Gew.% |
| 1,2-Dibrom-2,4-dicyanobutamin-2-phenoxyethanol | 0,1 Gew.% |
| Parfümöl | 0,1 Gew.% |
| Kreatin | 2,0 Gew.% |
| Wasser | ad 100,0 Gew.% |

| Beispiel 4: Haarkur | |
|---|---|
| Glycerinmonostearat | 6,0 Gew.% |
| Lanolinalkoxylat | 2,0 Gew.% |
| Cetylalkohol | 2,0 Gew.% |
| Gemisch aus Lanolinalkohol und Paraffinöl | 1,0 Gew.% |
| Tris-(oligooxyethyl)-alkyl-ammoniumphosphat | 1,5 Gew.% |
| Hydroxyethylcellulose | 20,0 Gew.% |
| Citronensäure | 0,1 Gew.% |
| Sorbinsäure | 0,1 Gew.% |
| Parfümöl | 0,1 Gew.% |
| Kreatin | 0,5 Gew.% |
| Wasser | ad 100,0 Gew.% |

| Beispiel 5: Schaumkonditioner | |
|---|---|
| PVP/Vinylimidazoliummethochlorid-Copolymer | 5,00 Gew.% |
| PVP/PVA-Copolymer | 1,00 Gew.% |
| Polyoxyethylen-12-cetylstearylalkohol | 0,15 Gew.% |
| Parfümöl | 0,10 Gew.% |
| Kreatin | 1,00 Gew.% |
| Propan/Butan | 10,00 Gew.% |
| Wasser | ad 100,00 Gew.% |

| Beispiel 6: Brillantine | |
|---|---|
| Candelillawachs | 80,0 Gew.% |
| Paraffinöl | 14,8 Gew.% |
| Isopropylmyristinat | 4,6 Gew.% |
| Parfümöl | 0,5 Gew.% |
| Kreatin | 0,1 Gew.% |
| | 100,0 Gew.% |

| Beispiel 7: Dauerwellmittel | |
|---|---|
| Thioglykolsäure (80%ige wässrige Lösung) | 9,5 Gew.% |
| Ammoniak (25% ige wässrige Lösung) | 1,6 Gew.% |
| Ammoniumcarbonat | 4,5 Gew.% |
| Kreatin | 2,0 Gew.% |
| Parfümöl | 0,2 Gew.% |
| Wasser | ad 100,0 Gew.% |

| Beispiel 8: Dauerwellfixierung | |
|---|---|
| Wasserstoffperoxid | 4,6 Gew.% |
| Citronensäure | 0,2 Gew.% |
| Kreatin | 3,0 Gew.% |
| Parfümöl | 0,1 Gew.% |
| Wasser | ad 100,0 Gew.% |

| Beispiel 9: Oxidationshaarfärbemittel in Cremeform | |
|---|---|
| Stearylalkohol | 8,00 Gew.% |
| Paraffinöl | 13,00 Gew.% |
| Wollfett | 6,00 Gew.% |
| Parfüm | 0,30 Gew.% |
| p-Toluylendiamin | 0,70 Gew.% |
| Resorcin | 0,05 Gew.% |
| Aminophenol | 0,06 Gew.% |
| Ethylendiamintetraacetat (EDTA) | 0,20 Gew.% |
| Ammoniak (25%ig ige wässrige Lösung) | 2,00 Gew.% |
| Natriumsulfit | 1,00 Gew.% |
| Kreatin | 1,00 Gew.% |
| Wasser | ad 100,00 Gew.% |

## Patentansprüche

1. Nicht-therapeutische Verwendung von Kreatin, Kreatinin und/oder deren Salzen zur Härtung, Stärkung und Restrukturierung von geschädigten menschlichen Haaren, wobei die Verwendung die Nasskämmbarkeit der Haare verbessert und die Bündelzugfestigkeit der Haare erhöht.

2. Verwendung nach Anspruch 1, **dadurch gekennzeichnet, dass** Kreatin oder Kreatinin und/oder dessen Salz ausgewählt ist aus Kreatinphosphat, Cyclokreatin, Phosphocyclokreatin und Kreatin-pyruvat.

3. Verwendung nach einem der Anspruche 1 oder 2, **dadurch gekennzeichnet, dass** das Kreatin, Kreatinin und/oder deren Salze vor, wahrend oder nach einer Exposition der Haare mit chemischen und/oder physikalischen Noxen mit den Haaren in Kontakt gebracht wird.

4. Verwendung nach Anspruch 3 in einem Vorbehandlungsmittel vor einer chemischen und/oder physikalischen Behandlung von menschlichen Haaren.

5. Verwendung nach einem der Anspruche 1 bis 4 zur Verhütung oder Verminderung von Schädigungen der inneren Struktur oder zur Reparatur (Restrukturierung) von menschlichen Haaren.

6. Verwendung nach einem der Anspruche 3 bis 5, **dadurch gekennzeichnet, dass** die chemische Behandlung eine Färbung, Tönung, Bleichung oder eine Dauerverformung umfasst.

7. Verwendung nach einem der Anspruche 1 bis 6, **dadurch gekennzeichnet, dass** das Kreatin, das Kreatinin und/oder deren Salze in einer Menge von 0,001 bis 30,0 Gewichtsprozent, bezogen auf die Gesamtmenge verwendet werden.

8. Verwendung nach einem der Anspruche 1 bis 7, **dadurch gekennzeichnet, dass** das Kreatin, das Kreatinin und/oder deren Salze in einer Menge von 0,05 bis 1,0 Gewichtsprozent, bezogen auf die Gesamtmenge verwendet werden.

9. Verwendung nach einem der Anspruche 1 bis 8, **dadurch gekennzeichnet, dass** das Kreatin, das Kreatinin und/oder deren Salze für eine Dauer von 1 bis 60 Minuten bei einer Temperatur zwischen 20°C und 60°C mit den menschlichen Haaren in Kontakt gebracht wird.

## Claims

1. A non-therapeutic use of creatine, creatinine, and/or the salts thereof for hardening, strengthening and restructuring damaged human hair, wherein the use improves the wet-combability of the hair and increases the tensile strength of the lock of hair.

2. The use according to Claim 1, **characterized in that** the creatine, creatinine, and/or the salts thereof is selected from creatine phosphate, cyclocreatine, phospho-cyclocreatine and creatine pyruvate.

3. The use according to either of Claims 1 or 2, **characterized in that** the creatine, creatinine, and/or the salts thereof is brought into contact with the hair before, during or after exposure of the hair to chemical and/or physical noxious substances.

4. The use according to Claim 3 in a pre-treatment agent before chemical and/or physical treatment of human hair.

5. The use according to any of Claims 1 through 4 to prevent or reduce damage to the inner structure or to repair (restructure) human hair.

6. The use according to any of Claims 3 through 5, **characterized in that** the chemical treatment comprises coloring, tinting, bleaching, or a permanent wave.

7. The use according to any of Claims 1 through 6, **characterized in that** the creatine, creatinine, and/or the salts thereof are used in a quantity of from 0.001 to 30.0 percent by weight, based on the total quantity.

8. The use according to any of Claims 1 through 7, **characterized in that** the creatine, creatinine, and/or the salts thereof are used in a quantity of from 0.05 to 1.0 percent by weight, based on the total quantity.

9. The use according to one of Claims 1 through 8, **characterized in that** the creatine, creatinine, and/or the salts thereof are brought into contact with human hair for a period of 1 to 60 minutes at a temperature of between 20°C and 60°C.

## Revendications

1. Utilisation non thérapeutique de créatine, créatinine et/ou de leurs sels pour le durcissement, le renforcement et la restructuration de cheveux humains abîmés, où l'utilisation améliore le coiffage des cheveux humides et augmente la résistance des mèches de cheveux à la traction.

2. Utilisation selon la revendication 1, **caractérisée en ce que** la créatine ou la créatinine et/ou le sel de celle-ci sont choisis parmi le phosphate de créatine, la cyclocréatine, la phosphocyclocréatine et le pyruvate de créatine.

3. Utilisation selon l'une quelconque des revendications 1 ou 2, **caractérisée en ce que** la créatine, la créatinine et/ou leurs sels sont mis en contact avec les cheveux avant, pendant ou après une exposition des cheveux à des agents nocifs physiques et/ou chimiques.

4. Utilisation selon la revendication 3, dans une composition de prétraitement avant un traitement physique et/ou chimique de cheveux humains.

5. Utilisation selon l'une quelconque des revendications 1 à 4, pour empêcher ou réduire des endommagements de la structure interne ou pour la réparation (restructuration) de cheveux humains.

6. Utilisation selon l'une quelconque des revendications 3 à 5, **caractérisée en ce que** le traitement chimique comprend une teinture, un nuançage, une décoloration ou une mise en forme permanente.

7. Utilisation selon l'une quelconque des revendications 1 à 6, **caractérisée en ce que** la créatine, la créatinine et/ou leurs sels sont utilisés en une quantité de 0,001 à 30,0 % en poids, par rapport à la quantité totale.

8. Utilisation selon l'une quelconque des revendications 1 à 7, **caractérisée en ce que** la créatine, la créatinine et/ou leurs sels sont utilisés en une quantité de 0,05 à 1,0 % en poids, par rapport à la quantité totale.

9. Utilisation selon l'une quelconque des revendications 1 à 8, **caractérisée en ce que** la créatine, la créatinine et/ou leurs sels sont mis en contact avec les cheveux humains pendant une durée de 1 à 60 minutes à une température comprise entre 20 °C et 60 °C.
